# EUROPEAN PATENT APPLICATION

(11) **EP 3 391 906 A1**
(43) Date of publication of application: **24.10.2018**
(21) Application number: 16875814.2
(22) Date of filing: 16.12.2016
(51) Int. Cl.: A61K 45/00, A61K 9/107, A61K 47/14, A61K 49/00, A61P 35/00

(54) **BIODEGRADABLE TUMOR SEALANT**

(30) Priority: 18.12.2015 JP 2015247650
(71) Applicant: Medigear International Corporation, Yokohama-shi, Kanagawa 226-8510 (JP); Tokyo Institute of Technology, Tokyo 152-8550 (JP); Farnex Incorporated, Ota-ku Tokyo 145-0064 (JP)
(72) Inventor: KONDOH, Shinae, Tokyo 152-8550 (JP); KUCHIMARU, Takahiro, Tokyo 152-8550 (JP); TANAKA, Takeo, Yokohama-shi Kanagawa 226-8510 (JP); ITAKURA, Shoko, Yokohama-shi Kanagawa 226-8510 (JP); HIJIKURO, Ichiro, Yokohama-shi Kanagawa 226-8510 (JP)
(74) Representative: AWA Sweden AB
(86) International application number: PCT/JP2016/087676
(87) International publication number: WO 2017/104840

(57) **Abstract**

The purpose of the invention is to provide a tumor sealant to be used for therapy that enables curative treatment, by improving an extremely simple, minimally-invasive therapy consisting of starving tumor cells without radiation exposure or causing serious side effects by drugs. The invention provides a tumor sealant that utilizes the EPR effect specific to tumor cells, that covers the tumor cells of each tissue by particles that permeate only tumor blood vessels and accumulate and settle in the tumor, that does not release angiogenesis-inducing factors, that does not separate tumor cells having improved mobility in a hypoxic state and move them to another location, that permeates gaps of from 50 nm to 500 nm of the tumor vascular endothelial cells, and that biodegrades after adhering to the tumor cells and extracellular matrix near the gaps.

## Description

### FIELD

The present invention relates to a particulate device that blocks and/or isolates tumor tissue from surrounding tissue containing blood vessels by surrounding and/or sealing the tumor by permeating blood vessels at the tumor site, selectively accumulate and/or adhere to the tumor tissue and settle for a fixed period of time, or a particulate device that blocks connections between blood vessels at the tumor site and tumor tissue.

### BACKGROUND

Trans-catheter arterial embolization (TAE) is a procedure used to treat lowly invasive liver cancer. Palliative care or terminal care is employed in cases in which liver damage is extensive and advanced to the degree that it is inoperable. This treatment method consists of embolizing the hepatic artery to interrupt the supply of nutrients and oxygen carried by the blood and eliminating the cancer by starvation. Although liver cells receive nutrients and so forth from the hepatic artery and portal vein, cancerous liver cells only receive supply of nutrients from the hepatic artery. On the other hand, since blood flow from the portal vein accounts for 70% of the blood flow in normal liver cells, normal cells are not considered to die off even following embolization of the hepatic artery.

In addition, when performing trans-catheter chemo-embolization (TACE), which combines trans-catheter arterial embolization with chemotherapy, the hepatic artery is embolized with an embolizing material after injecting a suspension of contrast agent and anticancer drug. At this time, since the suspension may flow into the hepatic vein, there is the risk of causing liver infarction due to embolization of the hepatic artery and portal vein. Moreover, there is also the possibility of cholecystitis and pancreatitis if the contrast agent, anticancer drug and embolizing material flow into the gallbladder or pancreas.

In consideration of this risk, embolizing materials have appeared that inhibit the sudden outflow of drugs and the like into blood vessels by providing with a function that enables spherical beads (micro spheres), which use a high molecular weight polymer for the base material and have a particle size of several hundred µm, instead of the previously described suspension, to be adsorbed onto an anticancer drug and then gradually released within an embolized vessel.

Embolizing materials consist of permanent embolizing materials that use a high molecular weight polymer as previously described for the raw material thereof and are permanently implanted in the body, and temporary embolizing materials that use a biodegradable substance such as starch or gelatin for the raw material and are degraded and metabolized in the body. However, many temporary embolizing materials do not demonstrate adequate embolization effects, and those substances derived from animals are prohibited from use by domestic academic societies.

On the other hand, risks associated with permanent implantation must be accepted in the case of permanent embolizing materials that demonstrate comparatively profound embolization effects. In cases in which inherent liver function has been lost as in cases of cirrhosis, metabolic disorders occur accompanying decreases in liver energy levels resulting in increased susceptibility to the occurrence of liver failure. Recanalization of blood flow to the hepatic artery and portal vein is required to minimize impairment of liver function as much as possible.

Therefore, PTL1 proposes an embolizing material that eliminates the occurrence of coagulative blockage in catheters and blood vessels other than the target vessel, completely embolizes a target site within a blood vessel, enables the embolized state to be released after a specific period of time, is degraded within the body as a result of being composed of a biodegradable substance that is metabolized or excreted outside the body.

In the case of arterial embolization (TAE/TACE), if a temporary embolizing material having an inadequate embolization effect is used or if an incompletely embolized state occurs due to the use of spherical embolization beads in which gaps form therein, not only are normal cells damaged due to inhibition of blood flow, but since this also causes the tumor environment to become hypoxic, there is an increased risk of this becoming a cause of malignant transformation.

According to PTL1, in a hypoxic state, even normal cells secrete protein that inhibits the function of immune cells of resisting cancer, and this is said to result in a portion of immune cells changing over to promoting cancer. It is described that, even if lowly malignant cancer cells are eliminated by a hypoxic state, highly malignant cancer cells that infiltrate and expand in tissue metastasize to other organs resulting in the formidable state of enhanced invasive capacity.

In general, it is extremely difficult for cancer treatment to only act on cancer tissue. In the case of surgical resection, normal tissue is resected over a wide range in order to avoid residual cancer cells. In the case of chemotherapy using anticancer drugs, normal tissue throughout the body is subjected to considerable damage due to intravenous infusion and injection. In the case of radiotherapy, although particle beams and other beams are narrowed, damage to normal tissue through which radiation passes cannot be avoided. Thus, there is a desire for a treatment method that enables only tumor tissue and tumor cells to be treated selectively.

As has been described above, although treatment methods using a microcatheter (TAE/TACE) are highly dependent on the skill of the physician, the nutrient artery of a tumor can be selectively embolized from among arteries in the vicinity of the tumor by using a microcatheter.

Contrivances have been made in chemotherapy, which consists of administration of an anticancer drug that allow the drug to be selectively delivered only to the tumor. One of these is referred to as active targeting, and consists of imparting a molecular target function demonstrated by a drug only to tumor cells with the aim of specifically targeting the tumor. In contrast, passive targeting is used as a method for delivering a drug based on a property or structure characteristic to tumor tissue or tumor cells.

A typical principle of passive targeting is the Enhanced Permeability and Retention (EPR) effect.

Although drugs and so forth do not leak from an intermediate point of blood vessels of normal tissue since they are tightly packed with endothelial cells, tumor blood vessels are structurally imperfect and there are said to be large gaps of 50 nm to 500 nm between endothelial cells. Microparticles having a particle size of 500 nm or less are able to permeate tumor blood vessels (i.e. demonstrate permeability).

In addition, since lymphatic vessels are undeveloped in tumor tissue, there is incomplete material circulation. Consequently, substances that pass through tumor blood vessels ultimately end up being retained and accumulate in the vicinity of the tumor (i.e. demonstrate retention).

Although extensive research has been conducted on drug delivery system (DDS) agents utilizing the aforementioned EPR effect for use in drug delivery, few cases of practical application are known. Even if a drug is delivered to the vicinity of a tumor, unless it is incorporated into the cells at the proper timing, its effect as a drug is diminished. Consequently, an even more elaborate mechanism is required for enabling drugs to permeate into cells.

Remarkable progress has been made in the area of medical materials in terms of such factors as superior biocompatibility, biodegradability and bio-environment responsivity, and artificial synthetic chemical materials have been developed that make it possible to avoid the risk of animal-derived materials.

In the area of amphiphilic compounds, which are known as artificial synthetic chemical materials that form liquid crystal, a low molecular weight liquid crystal compound has been reported that self-organizes into a cubic structure, inverse hexagonal structure or other non-lamellar structure having superior bioadhesion instead of a micelle structure, vesicle structure, dendrimer structure or other lamellar structure lacking in bioadhesion (PTL2, PTL3).

For example, PTL2 and PTL3 describe the development of a base material for injection (PTL2) and an adhesion preventive that utilizes bioadhesion (PTL3). However, these patent documents do not contain descriptions relating to an embolizing material, sealing material or application to embolization or sealing.

In addition, NPL2, PTL4 and PTL5 disclose embolizing materials for arterial embolization that use non-lamellar liquid crystal. However, the embolizing agents described in NPL2 and PTL4 to PTL6 use a mixed liquid of an amphiphilic compound and a water-soluble organic solvent and the like as a precursor of non-lamellar liquid crystal, and are emboli that control blood flow by forming a bulk liquid crystal gel within blood vessels or are limited to blood vessels that undergo embolization, and are not intended to directly accumulate in or seal tumor tissue through the use of the EPR effect.

### CITATION LIST

### PATENT LITERATURE

PTL1: JP-A 2004-313759
PTL2: WO 2011/078383
PTL3: WO 2014/178256
PTL4: Chinese Patent No. 103040741
PTL5: Chinese Patent No. 101822635
PTL6: Chinese Patent No. 103536974

### NON-PATENT LITERATURE

NPL1: Nikkei Science, Nov. 2014, R. K. Jain, "An Indirect Way to Tame Cancer", p. 61
NPL2: "Science Portal China", No. 48, New Drug Research and Development, Guanyi Chen, "Cubic Liquid Crystal in Controlled-Release Drugs", September 6, 2010

### SUMMARY

### TECHNICAL PROBLEM

An object of the present invention is to provide a tumor sealant that is lowly invasive, inhibits malignant transformation and enables cancer to be cured completely by a treatment method that starves tumor cells.

### SOLUTION TO PROBLEM

As a result of conductive extensive studies to solve the aforementioned problems, the inventors of the present invention found that tumor cells can be made to undergo necrosis by blocking the supply of nutrients and oxygen to a tumor and interrupting the transmission of induction factors from the tumor by allowing particles using a prescribed amphiphilic compound (lipid) as a base material to permeate the gaps between tumor vascular endothelial cells and adhere to extravascular tumor tissue, thereby leading to completion of the present invention.

Namely, the present invention includes that indicated below.
[1] A biodegradable tumor sealant containing microparticles having for the base material thereof a low molecular weight amphiphilic compound capable of forming non-lamellar liquid crystal.
[2] The biodegradable tumor sealant described in [1] above, wherein the microparticles have a particle size of 20 nm to 500 nm.
[3] The biodegradable tumor sealant described in [1] or [2] above, wherein the microparticles are present in the form of an emulsion dispersed in an aqueous medium.
[4] The biodegradable tumor sealant described in any of [1] to [3] above, wherein the amphiphilic compound is a compound represented by the following general formula (I) or a salt thereof. 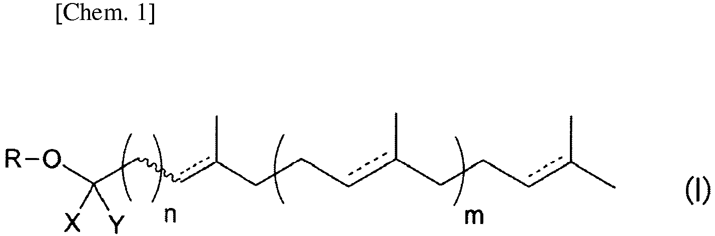 wherein, X and Y respectively represent a hydrogen atom or together represent an oxygen atom, n represents an integer of 0 to 2, and m represents 1 or 2, 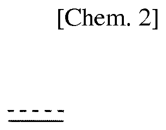 represents a single bond or double bond, and R represents a hydrophilic group having two or more hydroxyl groups.
[5] The biodegradable tumor sealant described in [4] above, wherein, in general formula (I), X and Y together represent an oxygen atom, and R represents a hydrophilic group obtained by removing a single hydroxyl group from glycerol.
[6] The biodegradable tumor sealant described in any of [1] to [5] above, wherein the amphiphilic compound is any of the following compounds: mono-O-(5,9,13-trimethyltetradecanoyl)glycerol, mono-O-(5,9,13,17-tetramethyloctadecanoyl)glycerol, mono-O-(5,9,13-trimethyltetradeca-4-enoyl)glycerol, mono-O-(5,9,13,17-tetramethyloctadeca-4-enoyl)glycerol, mono-O-(5,9,13-trimethyltetradeca-4,8,12-trienoyl)glycerol or mono-O-(5,9,13,17-tetramethyloctadeca-4,8,12,16-tetraenoyl)glycerol.
[7] The biodegradable tumor sealant described in any of [1] to [6] above, wherein the microparticles contain contrast agent and/or dye.
[8] The biodegradable tumor sealant described in [7] above, wherein the amphiphilic compound is mono-O-(5,9,13-trimethyltetradeca-4-enoyl)glycerol and the contrast agent is Lipiodol.

### ADVANTAGEOUS EFFECTS OF INVENTION

According to the present invention, solid malignant tumors can be treated by a treatment method that starves tumor cells in the absence of serious adverse side effects attributable to anticancer drugs and without causing significant trauma due to surgery.

### BRIEF DESCRIPTION OF DRAWINGS

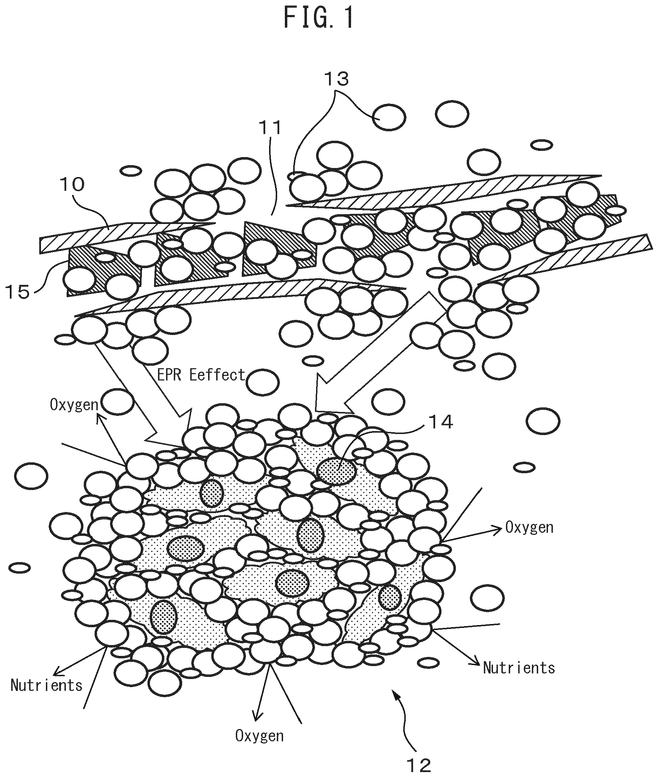

FIG. 1 is a drawing for explaining the concept of the present invention. FIG. 1 schematically shows microparticles that have permeated a tumor blood vessel by utilizing the EPR effect blocking the supply of nutrients and the like by surrounding the tumor.
FIG. 2(1) is a schematic diagram for explaining an emulsion that contains contrast agent-containing microparticles of the present invention in a first embodiment thereof. FIG. 2(2) is a schematic diagram for explaining the structure of the microparticles.
FIG. 3 is an intensity distribution diagram obtained during SAXS measurement relating to a gelatinous composition prepared in Example 2.
FIG. 4 depicts intensity distribution diagrams obtained during SAXS measurement relating to a gelatinous composition prepared in Example 3.
FIG. 5 is an intensity distribution diagram obtained during SAXS measurement relating to an emulsion prepared in Example 4.
FIG. 6 is a particle size distribution diagram obtained using dynamic light scattering relating to an emulsion prepared in Example 4.
FIG. 7 is an intensity distribution diagram obtained during SAXS measurement relating to an emulsion prepared in Example 5.
FIG. 8 is a particle size distribution diagram obtained using dynamic light scattering relating to an emulsion prepared in Example 5.
FIG. 9 is a particle size distribution diagram obtained using dynamic light scattering relating to an emulsion prepared in Example 7.
FIG. 10 depicts near infrared fluorescence images of a tumor-bearing mouse and an enlarged view of the tumor site.
FIG. 11 depicts CT images of the tumor site of a tumor-bearing mouse before and after administration of an emulsion prepared in Example 4.
FIG. 12 depicts CT images of a lung of a tumor-bearing mouse before administration, after a first administration and after a second administration of a 10-fold dilution of an emulsion prepared in Example 4.
FIG. 13 depicts captured images indicating the progression of degradation of a gel composition by mono-O-(5,9,13-trimethyltetradeca-4-enoyl)glycerol in human serum.
FIG. 14A indicates the effect of blood flow interruption at a tumor site of an emulsion based on breast cancer luciferase (Luc) activity. FIG. 14B is a graph plotting the size ratios of a tumor portion in which blood flow has been interrupted over time.
FIG. 15 depicts sectional images of time-based changes in blood oxygen concentration at a tumor site following administration of an emulsion. Blue color indicates reduced hemoglobin and red color indicates oxygenated hemoglobin.
FIG. 16A depicts tumor cross-sectional images obtained by measuring blood flow over time using indocyanine green (ICG). FIG. 16B is a graph indicating the results of recording changes in blood flow over time in the presence or absence of administration of an emulsion.
FIG. 17 indicates the results of observing changes in tumor volume over time in the case of having given multiple administrations of an emulsion.

### DESCRIPTION OF EMBODIMENTS

The following provides a detailed explanation of the present invention.

The biodegradable tumor sealant according to the present invention is comprised of microparticles that use as a base material thereof a low molecular weight amphiphilic compound capable of forming non-lamellar liquid crystal and have a particle size that enables the microparticles to permeate the gaps between tumor vascular endothelial cells, and are characterized by permeating the gaps followed by adhering to tumor tissue present in the vicinity of the gaps due to the high bioadhesiveness thereof and blocking the supply of nutrients and oxygen to the tumor as well as the transmission of induction factors from the tumor to cause the tumor cells to necrotize, after which the adhered microparticles are degraded and/or metabolized within the body. In addition, although the biodegradable tumor sealant according to the present invention has the aforementioned characteristics, since it also has the effect of reducing oxygen concentration and nutrient supply in the blood at the tumor site and necrotizing tumor cells by compressing nutrient vessels leading to the tumor and interrupting blood flow, the tumor sealant of the present invention also functions as a blood flow inhibitor having the aforementioned additional effects.

### 1. Amphiphilic Compound

The biodegradable tumor sealant according to the present invention contains a low molecular weight amphiphilic compound capable of forming non-lamellar liquid crystal. "Low molecular weight" in the present invention refers to having a molecular weight of about 20 to 10,000. Molecular weight is preferably 50 to 5,000, more preferably 100 to 2,500 and even more preferably 200 to 1,000. In addition, the "amphiphilic compound" of the present invention is an amphiphilic compound having a hydrophilic group and hydrophobic group that form water-in-oil non-lamellar liquid crystal in an aqueous medium.

Examples of the low molecular weight amphiphilic compound capable of forming non-lamellar liquid crystal include, but are not limited to, compounds represented by the following general formula (I): 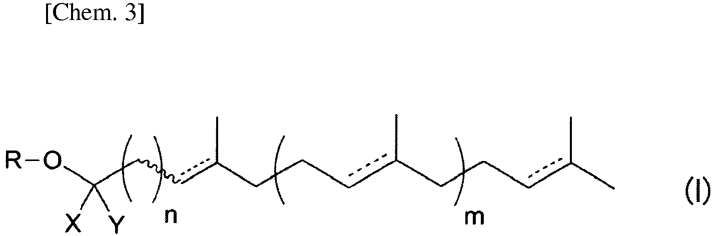 or a salt thereof.

In general formula (I), X and Y respectively represent a hydrogen atom or together represent an oxygen atom, and preferably together represent an oxygen atom.

n represents an integer of 0 to 2 and m represents 1 or 2. In the amphiphilic compound represented by general formula (I), the combination of n and m by any of n = 0 and m = 1, n = 0 and m = 2, n = 1 and m =1, n = 1 and m = 2, n = 2 and m = 1 or n = 2 and m = 2.

The notation: 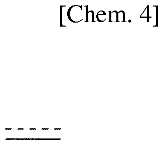 in the aforementioned general formula (I) represents a single bond or double bond.

Moreover, R in the aforementioned general formula (I) represents a hydrophilic group having two or more hydroxyl groups, and examples thereof include, but are not limited to, hydrophilic groups obtained by removing a single hydroxyl group from any of the following compounds selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol and xylitol. Preferable examples of R include hydrophilic groups obtained by removing a single hydroxyl group from glycerol.

In addition, in the present invention, the notation: 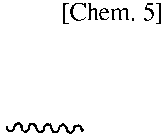 in general formula (I) means that the aforementioned amphiphilic compound is the E form (cis form), Z form (trans form) or mixture thereof of a geometric isomer thereof.

Preferable specific examples of the amphiphilic compound represented by general formula (I) include compounds in which X and Y together represent an oxygen atom and R represents a hydrophilic group obtained by removing a single hydroxyl group from glycerol.

Moreover, the amphiphilic compound represented by general formula (I) is preferably mono-O-(5,9,13-trimethyltetradecanoyl)glycerol, mono-O-(5,9,13,17-tetramethyloctadecanoyl)glycerol, mono-O-(5,9,13-trimethyltetradeca-4-enoyl)glycerol, mono-O-(5,9,13,17-tetramethyloctadeca-4-enoyl)glycerol, mono-O-(5,9,13-trimethyltetradeca-4,8,12-trienoyl)glycerol or mono-O-(5,9,13,17-tetramethyloctadeca-4,8,12,16-tetraenoyl)glycerol, and more preferably mono-O-(5,9,13-trimethyltetradeca-4-enoyl)glycerol.

The amphiphilic compound of the present invention can be synthesized with reference to the descriptions of the examples to be subsequently described. Alternatively, the amphiphilic compound of the present invention can be synthesized in accordance with the synthesis method described in, for example, WO 2014/178256, WO 2011/078383 or WO 2006/043705. The synthesized compound can be confirmed to have obtained the target compound by a routine method such as NMR.

### 2. Properties of Amphiphilic Compound

The amphiphilic compound used in the biodegradable tumor sealant according to the present invention is able to form non-lamellar liquid crystal in the form of a gelatinous composition by self-organizing in an aqueous medium. The non-lamellar liquid crystal formed by the amphiphilic compound used in the present invention has a structure that is not that of lamellar liquid crystal, and although it contains an L3 phase, it is preferably a type II (water-in-oil) liquid crystal in which hydrophobic groups are oriented towards the outside, and more specifically, is more preferably cubic liquid crystal or inverse hexagonal liquid crystal.

The aforementioned ability to form non-lamellar liquid crystal can be confirmed by analyzing the liquid crystal structure using ordinary methods. Type I (oil-in-water) cubic liquid crystal can be easily distinguished from type II (water-in-oil) cubic liquid crystal by the penetration method using a polarizing microscope. In addition, Pn3m cubic liquid crystal, Ia3d cubic liquid crystal, Im3m cubic liquid crystal and inverse hexagonal liquid crystal can be easily distinguished based on scattering peak ratios as determined by small-angle X-ray scattering (SAXS), and space group and lattice constant can be easily determined by determining the ratios of the reciprocals of peak values calculated from SAXS data.

Although there are no particular limitations thereon, examples of aqueous media enabling the amphiphilic compound according to the present invention to form non-lamellar liquid crystal include water such as sterilized water, purified water, distilled water, ion exchange water or ultrapure water, aqueous electrolyte solution such as physiological saline, aqueous sodium chloride solution, aqueous calcium chloride solution, aqueous magnesium chloride solution, aqueous sodium sulfate solution, aqueous potassium sulfate solution, aqueous sodium carbonate solution or aqueous sodium acetate solution, buffer solution such as phosphate buffer solution or Tris-HCl buffer solution, aqueous solution containing a water-soluble organic compound such as glycerin, ethylene glycol or ethanol, aqueous solution containing sugar molecules such as glucose, sucrose or maltose, aqueous solution containing a water-soluble polymer such as polyethylene glycol or polyvinyl alcohol, aqueous solution containing a surfactant such as octyl glucoside, dodecyl maltoside or Pluronic (polyethylene glycol/ polypropylene glycol/ polyethylene glycol copolymer), and body fluid such as intracellular fluid, extracellular fluid, interstitial fluid, lymph, spinal fluid, blood, gastric juice, serum, plasma, saliva, tears, semen or urine.

In particular, the amphiphilic compound represented by the aforementioned general formula (I) demonstrates high stability under a wide range of environmental conditions. For example, the amphiphilic compound according to the present invention is characterized by having a hydrophobic group in the form of an isoprenoid chain, and differing from amphiphilic compounds having a hydrophobic group in the form of oleic acid or other linear aliphatic chain, demonstrates high resistance to hydrolysis and high oxidation stability. The amphiphilic compound according to the present invention also has a wide temperature range over which it is able to form liquid crystal, has a low Krafft temperature, and is able to stably form liquid crystal even at low temperatures (6°C or lower and preferably 0°C or lower).

A noteworthy function of the non-lamellar liquid crystal formed by the amphiphilic compound used in the present invention is that it adheres to a surface of the body with two layers of phospholipids of the cell membrane and hydrophobic groups on the outside of the non-lamellar liquid crystal.

Moreover, another noteworthy function of the non-lamellar liquid crystal formed by the amphiphilic compound used in the present invention is the ability to incorporate a compound. For example, a hydrophobic compound can be stably incorporated on the inside of the liquid crystal, while a water-soluble compound can be stably incorporated in water channels constructed by the liquid crystal.

### 3. Preparation of Biodegradable Tumor Sealant

The biodegradable tumor sealant according to the present invention contains microparticles having a base material containing the aforementioned amphiphilic compound or is composed of those microparticles, and can be prepared in the form of an emulsion in which the aforementioned microparticles are dispersed in an aqueous medium.

Although there are no particular limitations thereon, examples of the aqueous medium for preparing the aforementioned microparticles in the form of an emulsion include water such as sterilized water, purified water, distilled water, ion exchange water or ultrapure water, aqueous electrolyte solution such as physiological saline, aqueous sodium chloride solution, aqueous calcium chloride solution, aqueous magnesium chloride solution, aqueous sodium sulfate solution, aqueous potassium sulfate solution, aqueous sodium carbonate solution or aqueous sodium acetate solution, buffer solution such as phosphate buffer solution or Tris-HCl buffer solution, aqueous solution containing a water-soluble organic compound such as glycerin, ethylene glycol or ethanol, aqueous solution containing sugar molecules such as glucose, sucrose or maltose, aqueous solution containing a water-soluble polymer such as polyethylene glycol or polyvinyl alcohol, aqueous solution containing a surfactant such as octyl glucoside, dodecyl maltoside or Pluronic (polyethylene glycol/ polypropylene glycol/ polyethylene glycol copolymer), and body fluid such as intracellular fluid, extracellular fluid, interstitial fluid, lymph, spinal fluid, blood, gastric juice, serum, plasma, saliva, tears, semen or urine.

Although the aforementioned amphiphilic compound may be contained alone for use as the base of the aforementioned microparticles, a functional substance may also be included. In terms of practical use, a contrast agent, dye or both is preferably contained in order to visualize the sealed state of the tumor sealant (and more specifically, the state of adherence and retention at a tumor site after having permeated a blood vessel, or the gradual biodegradation after having adhered thereto), In this manner, the tumor sealant of the present invention can be applied clinically to cancer diagnosis since it contains a contrast agent and/or dye.

Examples of contrast agents for X-ray CT include hydrophobic contrast agents in the form of iodinated contrast agent, Lipiodol and the aqueous contrast agent, iopamiron, examples of contrast agents for MRI include gadolinium (Gd)- or iron (Fe)-based magnetic substances, and examples of contrast agents for ultrasonography include ultrasonic liposome particles. One type or two or more types of contrast agents may be contained.

In addition, examples of dyes used to ensure visibility before or during surgery include fluorescent dyes such as hydrophobic coumarin, water-soluble fluorescein, pyranine or cyanine, and luminescent dyes such as luciferin. One type or two or more types of these dyes can be contained.

Moreover, one type or two or more types each of contrast agent and dye may also be contained.

The blending ratio between the aforementioned amphiphilic compound and the contrast agent and/or dye may be 100:1 to 1:100, and, for example, can be 95:5, 10:1, 90:10, 10:2, 80:20, 10:3, 70:30, 60:40, 50:50, 40:60, 30:70, 20:80, 10:90 or 5:95, and is preferably 100:1 to 50:50 more preferably 10:1 to 10:3.

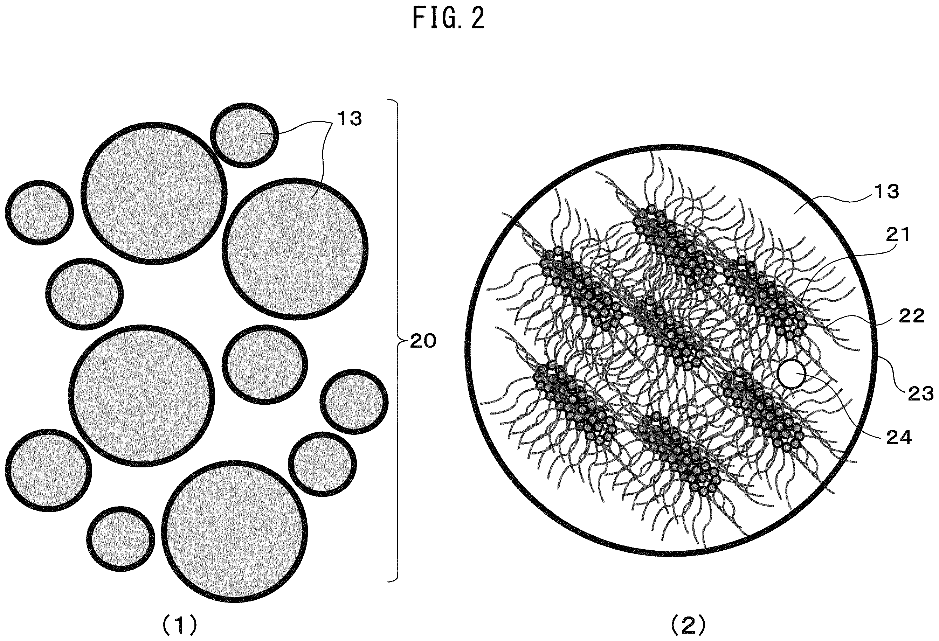

Fig. 2(1) schematically shows an emulsion 20 that composes a biodegradable tumor sealant and contains microparticles 13 of various particle sizes. Since the microparticles 13 are formed by shredding of non-lamellar liquid crystal to be subsequently described, although the particle size thereof is not uniform and the microparticles 13 consist of a mixture of various sizes as shown in Fig. 2(1), a particle size of 20 nm to 500 nm is predominant (preferably accounting for 70% or more of all microparticles, more preferably accounting for 90% or more, and potentially accounting for 100% depending on the case), while microparticles 13 having a particle size of less than 20 nm or microparticles 13 having a particle size in excess of 500 nm can also be present. Thus, in the present description, in the case of specifying the microparticles 13 according to particle size corresponding to the form of use, microparticles having a particle size of "20 nm to 500 nm" are not intended to exclude microparticles having a particle size of, for example, "10 nm to 200 nm" or microparticles having a particle size of "300 nm to 700 nm". On the other hand, a particle size of "20 nm to 500 nm" is intended to exemplarily include particle sizes such as "20 nm to 400 nm", "20 nm to 300 nm", "20 nm to 200 nm", "20 nm to 100 nm", "30 nm to 500 nm", "30 nm to 400 nm", "30 nm to 300 nm", "30 nm to 200 nm", "30 nm to 100 nm", "40 nm to 500 nm", "40 nm to 400 nm", "40 nm to 300 nm", "40 nm to 200 nm", "40 nm to 100 nm", "50 nm to 500 nm", "50 nm to 400 nm", "50 nm to 300 nm", "50 nm to 200 nm", "50 nm to 100 nm", "100 nm to 500 nm", "100 nm to 400 nm", "100 nm to 300 nm" or "100 nm to 200 nm".

The aforementioned microparticles can typically be prepared by adding one type or two or more types of the aforementioned amphiphilic compound or salt thereof, contrast agent and/or dye (in the case of the inclusion thereof), a suitable amount of one or two or more types of a pharmaceutically acceptable surfactant, and a suitable amount of an aqueous medium (such as physiological saline or water for injection) in any arbitrary order followed by stirring and homogenization. The case in which the inner phase of the microparticles consists of inverse hexagonal liquid crystal and the contrast agent and/or dye is hydrophobic is shown in the microparticles 13 of Fig. 2(2). Namely, a hydrophobic contrast agent and/or dye 24 are contained on the side of the liquid crystal structure of inverse hexagonal liquid crystal 21. Conversely, a water-soluble contrast agent and/or dye 24 are contained in a water channel 22. Furthermore, the microparticles 13 are able to remain stable in an aqueous medium by covering with an outer phase 23 containing a surfactant.

Any arbitrary surfactant used in the field of pharmaceuticals or cosmetics can be used for the aforementioned pharmaceutically acceptable surfactant, and although not limited to those indicated below, examples thereof which can be used include Pluronic (such as Pluronic F127: polyoxyethylene polyoxypropylene (200 EO) (70 PO) and Polysorbate 80 (polyoxyethylene sorbitan monooleate: Tween 80).

In one embodiment thereof, although the biodegradable tumor sealant of the present invention may contain the aforementioned microparticles at a concentration that allows the microparticles to be dispersed therein (such as 0.001% by weight to 15% by weight), the concentration thereof is determined according to such factors as the administration target or dose.

The aforementioned microparticles can be confirmed by analyzing the structure of the microparticles by, for example, small-angle X-ray scattering (SAXS) or cryogenic transmission electron microscopy (cryo-TEM). In addition, particle size distribution of the microparticles can be measured with, for example, a zeta potential particle size analyzer.

As was indicated in the aforementioned typical example thereof, the although the microparticles of the present invention can be prepared by mixing one or more types of amphiphilic compounds with a surfactant and the like followed by stirring and homogenization, as indicated in Example 10 to be subsequently described, the microparticles can also be prepared by using ultrasonic disintegration or microfluidics. Here, "microfluidics" is the generic term for a technology involving the behavior of fluids in a microscopic space and devices using this technology are referred to as "microfluidic devices". These devices have channel structures typically having a depth and width of about several micrometers to several hundred micrometers that are fabricated by applying semiconductor microprocessing technology and precision machining technology. In the present description, although microparticles are described as having been fabricated using microfluidics technology, the microparticles are preferably fabricated using this technology since microparticles having a desired particle size can be stably prepared. Furthermore, microparticle size can be measured using a common method widely known among persons with ordinary skill in the art.

### 4. Properties and Characteristics of Biodegradable Tumor Sealant

### (1) Syringe or Microcatheter Passage

As was previously described, the biodegradable tumor sealant according to the present invention contains an emulsion obtained by dispersing microparticles of non-lamellar liquid crystal formed by the aforementioned amphiphilic compound in a biocompatible aqueous medium. This emulsion ensures passage through a syringe or microcatheter.

### (2) Tumor Vascular Permeability

Since newly formed tumor blood vessels specifically formed in tumor tissue have reduced levels of pericytes that cover blood vessels, the gaps between vascular endothelial cells in the tumor vessel are larger in comparison with those of normal cells. There are particles that are able to escape through these gaps. The present biodegradable tumor sealant has a particle size sufficiently small for passing through these gaps. Although varying according to the individual and according to the type and site of the tumor, the size of these gaps is typically 5 nm to 700 nm.

### (3) Dispersibility Composed of Different Particle Sizes

The gaps in the aforementioned tumor vessel vary in size. Since the present biodegradable tumor sealant is in the form of an emulsion in which particle size is not uniform but rather distributed over a range of several hundred nanometers, integration density can be increased regardless of the gap through which the microparticles pass.

### (4) Tumor Tissue Accumulativity

Although microparticles that have passed through a tumor vessel in the manner described above are released in the vicinity of the tumor, differing from normal tissue, the lymphatic network is not developed in tumor tissue. Consequently, the microparticles are not transported to other sites together with body fluid via lymphatic vessels. Thus, the released microparticles, or in other words, the present biodegradable tumor sealant has accumulativity that enables it to be retained at the tumor site. The aforementioned (2) and (4) are collectively referred to as the EPR effect.

### (5) Tumor Tissue Settleability

Even if the microparticles accumulate in tumor tissue as previously described, they end up flowing away with the flow of body fluids and the like unless they settle in tumor cells and interstitium. Since the present biodegradable tumor sealant is a substance having a liquid crystal structure that is not a lamellar structure, it demonstrates high adhesiveness to the cell membrane and extracellular matrix composed of collagen, fibroblasts and the like, thereby having superior settleability.

### (6) Blockage of Oxygen and Nutrients

As a result of the present biodegradable tumor sealant gathering and coalescing, enabling the gelatinous tumor sealant to effectively seal tumor tissue, the supply of nutrients and oxygen required for the growth of tumor cells is blocked, thereby leading to cell death (necrosis).

### (7) Inhibition of Transmission of Transmitted Transcription Induction Factors

Various types of induction factors are emitted from tumor tissue and cells. For example, hypoxia inducible factor (HIF), which is emitted from cancer cells under hypoxic stress, is transmitted to vascular endothelial growth factor (VEGF) and promotes tumor vascularization, thereby contributing to growth by increasing the amounts of nutrients and oxygen supplied to the tumor. The present biodegradable tumor sealant is able to prevent malignant transformation by inhibiting the diffusion of these induction factors emitted from tumors by continuously covering and entrapping tumor tissue.

### (8) Rapid Metabolism and Excretion

The present biodegradable tumor sealant is degraded or solubilized by enzymes in the body, and a portion thereof undergo further degradation followed by being excreted outside the body, after an adequate amount of time or time period has elapsed for causing the tumor cells to undergo necrosis.

### (9) Visibility and Visualization

Although the properties of the present biodegradable tumor sealant are limited to the basic functions described through section (6), functions used by physicians that are useful in the clinical setting are added as functions for practical use.

The first such function is visibility through visual observation. As a result of containing a dye (including fluorescent dye), the risk of being mistaken for other pharmaceuticals can be reduced, and by indicating a surgical site or range when used as a marker, application to an incorrect location can be avoided.

Secondly, by containing a contrast agent, whether or not the tumor sealant is applied or has been applied to the proper location can be confirmed by monitoring with an imaging device during surgery. Moreover, since the tumor sealant settles at the tumor site without running out as occurs when using a contrast agent alone, the tumor sealant can be visualized by allowing postoperative tumor status to be monitored with an imaging device without having to undergo a procedure for re-injecting the contrast agent.

### (10) Embolization

The present biodegradable tumor sealant can also be used as embolizing material for arterial embolization. The use of a temporary embolizing material, which demonstrates embolization effects comparable to those of permanent embolizing materials, for the embolizing material makes it possible to avoid the risk of permanent implantation, while also making it possible to anticipate synergistic effects of the tumor sealing effect and embolization effect.

### 5. Use of Biodegradable Tumor Sealant

The biodegradable tumor sealant prepared as previously described can be used to diagnose and/or treat malignant solid tumors. When used in the present description, "malignant solid tumors" include squamous cell carcinoma, breast cancer, cutaneous lymphoma, angiosarcoma, hepatobiliary cancer, head and neck cancer, lung cancer, mesothelioma, mediastinal carcinoma, esophageal cancer, stomach cancer, pancreatic cancer, small intestinal cancer, colon cancer, colorectal cancer, large bowel cancer, anal cancer, kidney cancer, urethral cancer, bladder cancer, prostate cancer, urethral cancer, penile cancer, testicular cancer, gynecological organ cancer, ovarian cancer, endocrine cancer, skin cancer, cancer of the central nervous system including the brain, soft tissue cancer, osteosarcoma, and melanoma originating in the skin and eye. Although there are no limitations thereon, the form of use of the biodegradable tumor sealant may be in the form of an emulsion in which microparticles containing one type or two or more types of an amphiphilic compound for the base material thereof are dispersed in an aqueous medium, or in the form of a pharmaceutical composition containing these microparticles. In this manner, not only is the tumor sealant of the present invention able to be applied to the diagnosis of malignant solid tumors and other cancer, but since it can also be applied to cancer treatment, it can be referred to as a "therapeutic device".

According to the present invention, the biodegradable tumor sealant may be introduced using a syringe or microcatheter so as to embolize or inhibit blood flow to a tumor from an artery in the vicinity of the tumor. In one aspect thereof, the biodegradable tumor sealant of the present invention can be locally administered in the vicinity of a tumor using a syringe, although not limited thereto. Here, the amount, concentration, number of administrations and administration frequency of the biodegradable tumor sealant at the time of introduction can be suitably adjusted by a physician or veterinarian in consideration of the gender, age, body weight and lesion status of the patient (subject). Here, the treatment target of the biodegradable tumor sealant of the present invention is preferably a mammal, and although there are no limitations thereon, examples of mammals include humans, monkeys and other primates, mice, rats, rabbits, guinea pigs and other rodents, cats, dogs, sheep, pigs, cows, horses, donkeys, goats and ferrets.

In Example 8 to be subsequently described, an antitumor effect was obtained by administering (and more specifically, injecting) 100 µL of the biodegradable tumor sealant in the form of an emulsion (Example 4) into a blood vessel of tumor-bearing mice in the vicinity of the tumor with a syringe. With respect to application to humans, this is equivalent to administering the biodegradable tumor sealant of the present invention in an amount of about 10 ml to 200 ml per administration, when converting simply on the basis of body weight, into a blood vessel in the vicinity of a tumor based on experimental results using these tumor-bearing mice. In addition, the biodegradable tumor sealant of the present invention may be administered several times (such as two to ten times) at suitable intervals (such as twice per day, once per day, twice per week, once per week or once every two weeks) until the desired therapeutic effect is obtained. Furthermore, the dose (and more specifically, the injected amount) per administration and administration frequency of the biodegradable tumor sealant of the present invention are not limited to that described above, but rather can be suitably adjusted and determined by a person with ordinary skill in the art (such as a physician or veterinarian).

In the case of using the biodegradable tumor sealant of the present invention as a pharmaceutical composition, the pharmaceutical composition may be used in a form in which a carrier, vehicle and/or stabilizer and the like are suitably added in addition to the biodegradable tumor sealant contained as an active ingredient. In addition, according to the present invention, the pharmaceutical composition is supplied in the form of a kit containing containers (such as vials) respectively enclosing the biodegradable tumor sealant, solvent (such as physiological saline) and carrier and the like along with instructions for use. In addition, the biodegradable tumor sealant of the present invention includes medical materials.

### EXAMPLES

The following provides a more detailed explanation of the present invention based on examples thereof. The present invention is naturally not limited to the following examples.

### [Example 1] Synthesis of Mono-O-(5,9,13-trimethyltetradeca-4-enoyl)glycerol

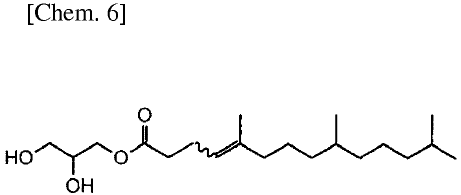

1.0 g (3.5 mmol) of methyl 5,9,13-trimethyltetradeca-4-enoate were slowly dropped into a dry N,N-dimethylformamide solution (3.5 mL) of 0.65 g (7.1 mmol) of glycerol and 0.59 g (4.3 mmol) of potassium carbonate. After stirring for 18 hours at 100°C, 1 M hydrochloric acid was added to the reaction liquid followed by extraction with ether. The extract was sequentially washed with saturated sodium bicarbonate and saturated saltwater, dried with anhydrous sodium sulfate, filtered and concentrated. The resulting residue was purified by silica gel column chromatography (mixture of ethyl acetate and hexane) to obtain the target compound in the form of a colorless, clear liquid.

The results of measuring the ¹H-NMR spectrum and viscosity of the resulting compound were as shown below.
¹H-NMR spectrum (300 MHz, CDCl3, TMS) δ: 0.80-0.90 (m,9H), 1.00-1.70 (m,15H), 1.97 (td, J=7.8, 17.0 Hz, 2H), 2.13 (t, J=6.1 Hz, 1H, OH), 2.25-2.45 (m,4H), 2.55 (d, J=5.2 Hz, 1H, OH), 3.50.4.00 (m,3H), 4.10-4.25 (m,2H), 5.08 (t, J=6.7 Hz, 1H)
Viscosity: 0.48 Pa·s (shear rate: 92 1/s)

### [Example 2] Formation and Analysis of Liquid Crystal by

### Mono-O-(5,9,13-trimethyltetradeca-4-enoyl)glycerol

An aqueous medium in the form of a 50% by weight or more of physiological saline was added to mono-O-(5,9,13-trimethyltetradeca-4-enoyl)glycerol followed by carrying out a mixing procedure and removing the separated physiological saline. As a result, a composition was obtained having the appearance of a cloudy to colorless, clear gel.

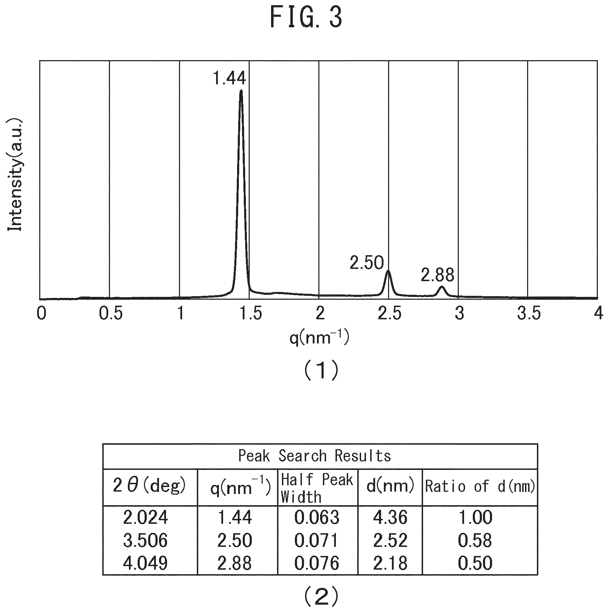

The liquid crystal structure of the aforementioned gelatinous composition was analyzed by small-angle X-ray scattering (SAXS) using the NANO Viewer nanoscale X-ray structural evaluation system (Rigaku). As a result of measuring the aforementioned gelatinous composition packed into pinhole slits under atmospheric pressure, a graph of SAXS intensity distribution (Fig. 3(1)) and peak search results (Fig. 3(2)) were obtained. As a result, at least three scattering peaks were observed, and since the ratio of the peaks was a ratio of 1:√3:2, which is characteristic to inverse hexagonal liquid crystal, the aforementioned gelatinous composition was able to be confirmed to be inverse hexagonal liquid crystal.

### [Example 3] Formation and Analysis of Liquid Crystal by

### Mono-O-(5,9,13-trimethyltetradeca-4-enoyl)glycerol and Lipiodol

Mono-O-(5,9,13-trimethyltetradeca-4-enoyl)glycerol and a contrast agent in the form of Lipiodol were mixed at mixing ratios of 10:5, 70:30, 10:3, 80:20, 10:2, 90:10 and 10:1. An aqueous medium in the form of 50% by weight or more of physiological saline was added to each mixture followed by carrying out a mixing procedure and removing the separated physiological saline to respectively obtain mixtures. The mixtures mixed at mixing ratios of 10:5 and 70:30 were compositions that separated into two layers. The mixtures mixed at mixing ratios of 10:3, 80:20, 10:2, 90:10 and 10:1 each became a cloudy gelatinous composition, and viscosity increased as the weight ratio of Lipiodol became lower.

The liquid crystal structures of the aforementioned gelatinous compositions were analyzed by small-angle X-ray scattering (SAXS) using the SmartLab high-luminance in-plane X-ray diffraction system (Rigaku). The aforementioned gelatinous composition packed into pinhole slits were measured under atmospheric pressure.

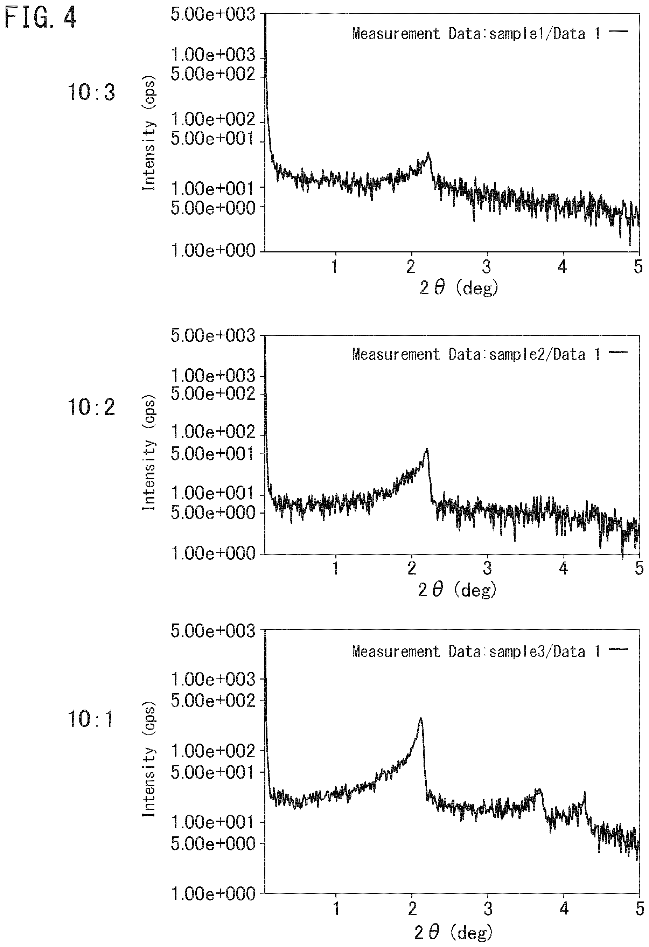

Fig. 4 depicts graphs of the results of SAXS measurement in the case of a weight ratio of 10:3 (Fig. 4(1)), 10:2 (Fig. 4(2)) and 10:1 (Fig. 4(3)). All three samples were observed to form non-lamellar liquid crystal. In contrast to three intense scattering peaks being observed at the characteristic ratio of inverse hexagonal liquid crystal in the case of a weight ratio of 10:1, the three scattering peaks diminished as the weight ratio changed to 10:2 and 10:3 and the regularity of the crystal structure decreased.

### [Example 4] Preparation of Emulsion Consisting of

### Mono-O-(5,9,13-trimethyltetradeca-4-enoyl)glycerol and Lipiodol

1.841 g of mono-O-(5,9,13-trimethyltetradeca-4-enoyl)glycerol and 0.184 g of a contrast agent in the form of Lipiodol were mixed so that the weight ratio of mono-O-(5,9,13-trimethyltetradeca-4-enoyl)glycerol to Lipiodol was 10:1 followed by the addition of 0.51 g of Pluronic F127 (Aldrich P2443) and 0.285 g of ethanol and dissolving to uniformity. Moreover, after adding 12.18 g of physiological saline (Otsuka Pharmaceutical) and stirring, an emulsion containing microparticles was obtained by homogenizing with a Star Burst Homogenizer (Sugino Machine).

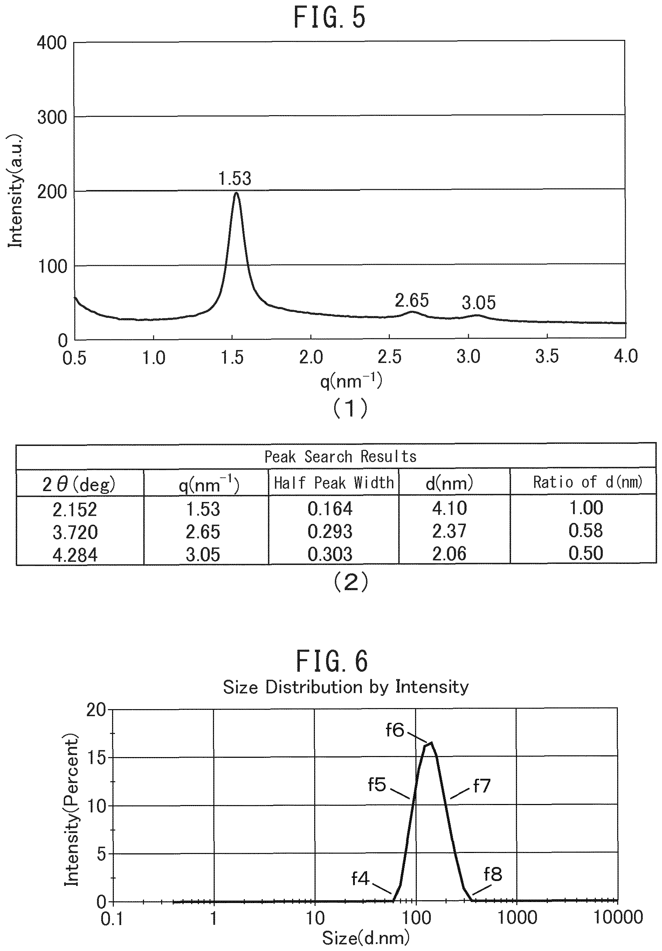

Liquid crystal structure of the aforementioned emulsion was analyzed by small-angle X-ray scattering (SAXS) using the NANO Viewer nanoscale X-ray structural evaluation system (Rigaku). As a result of introducing the emulsion into a capillary tube under atmospheric pressure and measuring with the system at reduced pressure (although the sample per se was at atmospheric pressure), a graph of SAXS intensity distribution (Fig. 5(1)) and peak search results (Fig. 5(2)) were obtained. As a result, at least three scattering peaks were observed, and since the ratio of the peaks was a ratio of 1: √3:2, which is characteristic to inverse hexagonal liquid crystal, the aforementioned emulsion was indicated to be a liquid crystal emulsion in which microparticles of inverse hexagonal liquid crystal were dispersed.

Moreover, particle size distribution of the aforementioned emulsion was measured using the Zeta Sizer Nano-ZS (Malvern). The measurement sample was prepared by diluting the aforementioned emulsion 100-fold with distilled water. The resulting measurement data is shown in Fig. 6. Average particle size (Z-average) was 131.4 nm. In Fig. 6, microparticles having a particle size in the vicinity of 60 nm began to be formed and particle size distribution increased rapidly as particle size increased, eventually reaching a peak in the vicinity of a particle size of 130 nm. Subsequently, formation of microparticles concluded in the vicinity of a particle size of 350 nm while particle size distribution decreased as particle size increased. Namely, the majority of the aforementioned emulsion consisted of microparticles having a particle size of 60 nm to 350 nm.

### [Example 5] Preparation of Emulsion of

### Mono-O-(5,9,13-trimethyltetradeca-4-enoyl)glycerol

An emulsion containing microparticles was prepared by using 5.4 g of mono-O-(5,9,13-trimethyltetradeca-4-enoyl)glycerol, 1.36 g of Pluronic F127 (Aldrich P2443), 0.76 g of ethanol and 32.48 g of water for injection (Otsuka Pharmaceutical) followed by stirring and homogenizing in the same manner as Example 4.

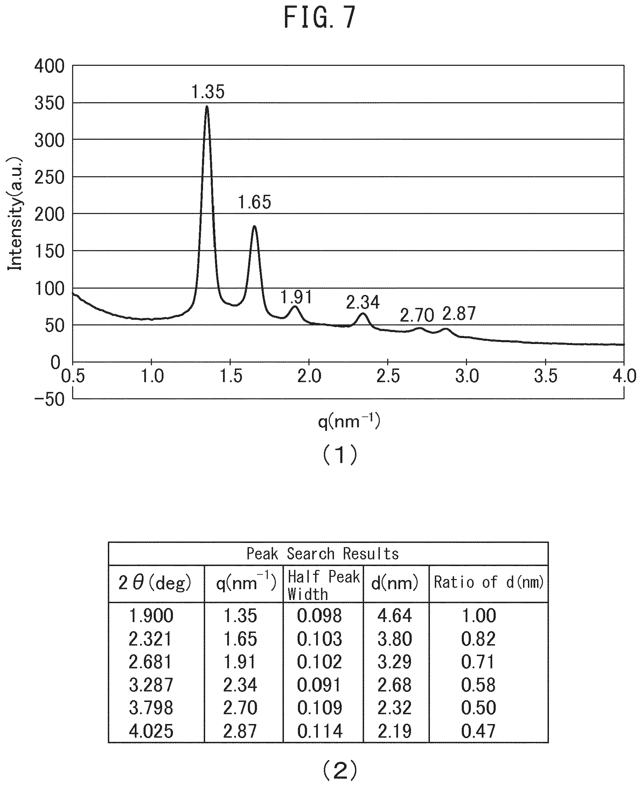

The aforementioned emulsion was measured by SAXS in the same manner as Example 4. As a result, a graph of SAXS intensity distribution (Fig. 7(1)) and peak search results (Fig. 7(2)) were obtained. As a result, at least six scattering peaks were observed, and since the ratio of the peaks was a ratio of √2: √3: √4: √6: √8: √9, which is characteristic cubic liquid crystal belonging to the crystallographic space group Pn3m, the aforementioned emulsion was indicated to be a liquid crystal emulsion of cubic liquid crystal belonging to the crystallographic space group Pn3m (cubosome).

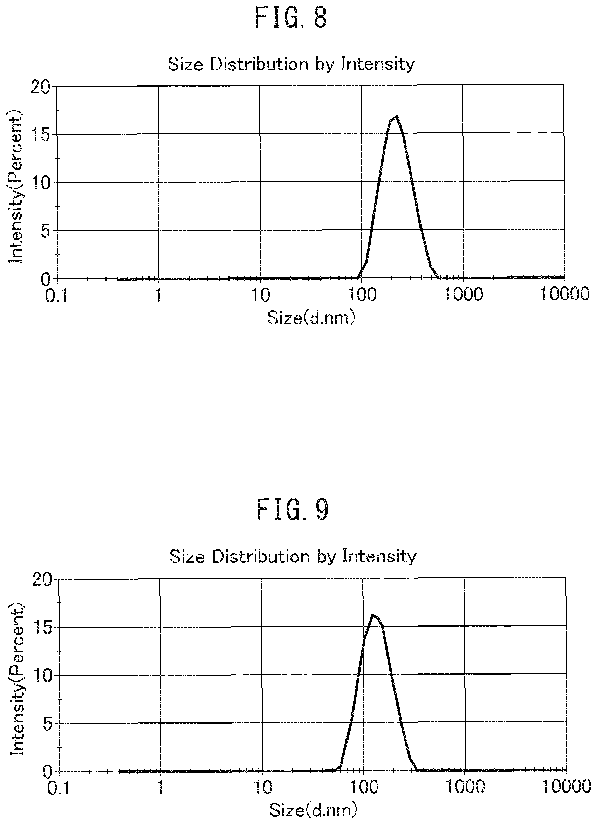

Moreover, particle size distribution of the aforementioned emulsion was measured in the same manner as Example 4. The measurement sample was prepared by diluting the aforementioned emulsion 100-fold with distilled water. The results are shown in Fig. 8. Average particle size (Z-average) of the aforementioned emulsion was 208.8 nm, and the majority of the aforementioned emulsion consisted of microparticles having a particle size of 90 nm to 600 nm.

### [Example 6] Preparation of Emulsion of

### Mono-O-(5,9,13-trimethyltetradeca-4-enoyl)glycerol and Cy7 Carboxylic Acid

An emulsion containing microparticles was prepared by using 3.375 g of mono-O-(5,9,13-trimethyltetradeca-4-enoyl)glycerol, 0.0074 g of a fluorescent dye in the form of Cy7 carboxylic acid (Lumiprobe-0550-90, Lumiprobe), 0.85 g of Pluronic F127 (Aldrich P2443), 0.475g of ethanol and 20.3 g of physiological saline (Otsuka Pharmaceutical) followed by stirring and homogenizing in the same manner as Example 4.

### [Example 7] Preparation of Emulsion of Mono-O-(5,9,13-trimethyltetradeca-4-enoyl)glycerol, Lipiodol and Fluorescein Sodium

An emulsion containing microparticles was prepared by using 2.4546 g of mono-O-(5,9,13-trimethyltetradeca-4-enoyl)glycerol, 0.2454 g of a contrast agent in the form of Lipiodol, 0.0037 g of a fluorescent dye in the form fluorescein sodium (uranine), 0.68 g of Pluronic F127 (Aldrich P2443), 0.38 g of ethanol and 16.2363 g of physiological saline (Otsuka Pharmaceutical) followed by stirring and homogenizing in the same manner as Example 4.

Particle size distribution of the aforementioned emulsion was measured in the same manner as Example 4. The measurement sample was prepared by diluting the aforementioned emulsion 100-fold with distilled water. The results are shown in Fig. 9. Average particle size (Z-average) of the aforementioned emulsion was 124.8 nm, and the majority of the aforementioned emulsion consisted of microparticles having a particle size of 60 nm to 300 nm.

### [Example 8] Tumor Sealing Effect using Emulsion and Evaluation of Effect on Normal Tissue

### (1) Production of Tumor-Bearing Mouse

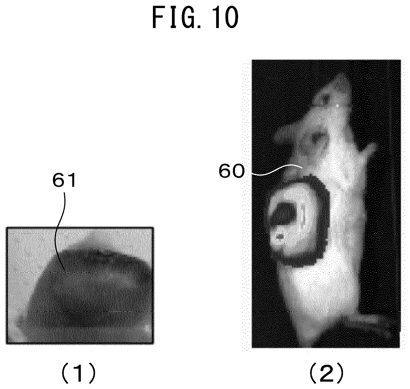

Mouse lung carcinoma (LLC) constitutively expressing firefly luciferase gene (luc) (LLC/luc) was transplanted to a site located 1 mm beneath the skin in the middle of the back of a laboratory mouse (C57BL/6 albino, male). A ten-week-old tumor-bearing mouse (body weight: 26.2 g) by allowing the tumor to grow over the course of 36 days. Fig. 10 depicts images of the tumor-bearing mouse and the tumor site visualized by bioluminescence with luciferase.

### (2) Experiment on Tumor Sealing Effect

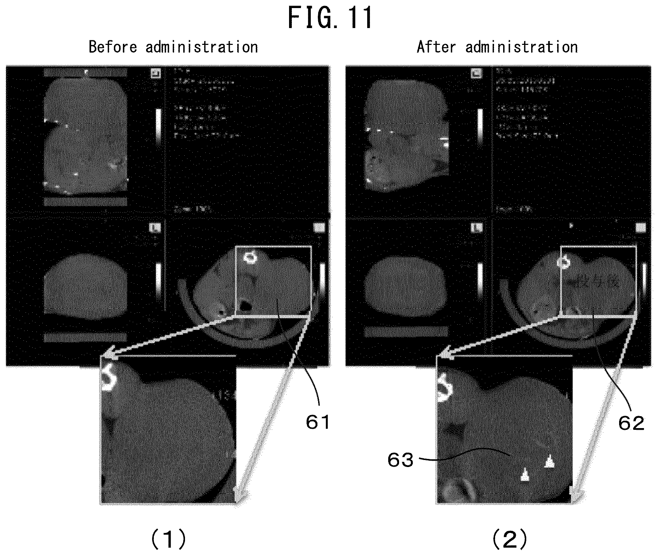

100 µL of the emulsion prepared in Example 4 were administered to the tumor of the aforementioned tumor-bearing mouse through a blood vessel in the vicinity of the tumor with a syringe. The tumor site was observed before administration and five minutes after administration using a small laboratory animal 3D Micro X-ray CT system (CosmoScan FX, Rigaku). As a result of analyzing the resulting CT images, the microparticles in the emulsion were clearly determined to have permeated the tumor blood vessel and adhered to the tumor site. Fig. 11 depicts CT images of the tumor site of the tumor-bearing mouse before and after administration of the present emulsion.

### (3) Experiment on Effect on Normal Tissue

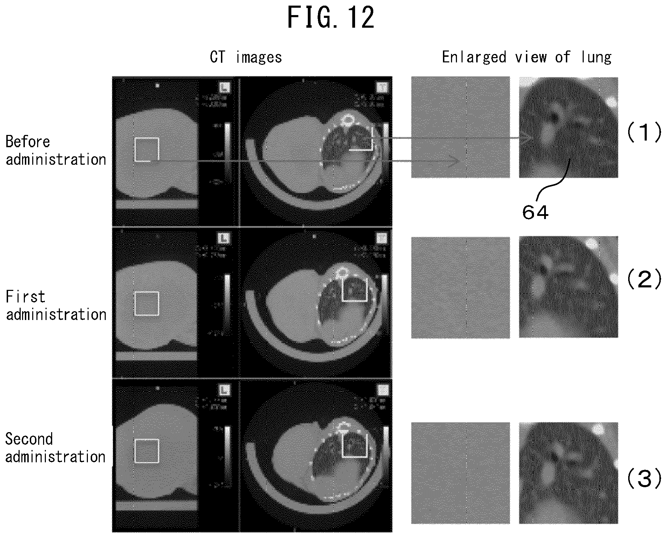

200 µL of an emulsion obtained by diluting the emulsion prepared in Example 4 10-fold with physiological saline was administered twice into the tail of the aforementioned tumor-bearing mouse every 10 minutes with a syringe. The entire tumor-bearing mouse was observed before administration, 5 minutes after the first administration and 5 minutes after the second administration using a small laboratory animal 3D Micro X-ray CT system (CosmoScan FX, Rigaku). Microparticles in the emulsion were clearly determined to have not adhered to normal tissue either after the first administration or after the second administration particularly in the lungs where the narrowest capillaries are present. Fig. 12 depicts CT images of the lungs of the tumor-bearing mouse before administration, after the first administration and after the second administration of the present emulsion.

According the above results, the microparticles (biodegradable tumor sealant) of the present invention were suggested to specifically adhere to tumor cells but not adhere to normal tissue.

### [Example 9] Liquid Crystal Obtained from

### Mono-O-(5,9,13-trimethyltetradeca-4-enoyl)glycerol and Evaluation of Stability of that Liquid Crystal containing Lipiodol in Human Serum

Approximately 100 µL of mono-O-(5,9,13-trimethyltetradeca-4-enoyl)glycerol or approximately 100 µL each of mixed solutions of mono-O-(5,9,13-trimethyltetradeca-4-enoyl)glycerol and Lipiodol at a weight ratio of 10:1, 10:2 or 10:3 were respectively added to vials containing 2 mL of human serum (Human OTC Serum, Access Biologicals). After gently shaking each vial to cause the formation of a gelatinous composition, the vials were allowed to stand undisturbed in a water bath at 37°C followed by observing immediately thereafter, 10 hours later and 24 hours later. The human serum in each vial gradually became cloudy with the passage of time. Furthermore, in the case of having used distilled water instead of human serum, there was no change in the gelatinous composition and there was hardly any cloudiness in the distilled water in any of the samples within 24 hours.

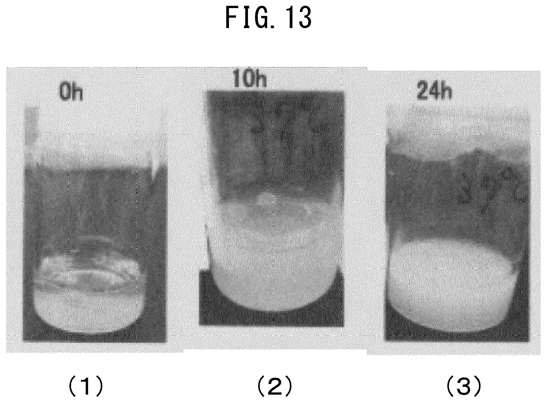

Fig. 13 depicts captured images indicating changes over time in samples consisting of approximately 100 µL of mono-O-(5,9,13-trimethyltetradeca-4-enoyl)glycerol. Immediately after addition in the human serum (Fig. 13(1), a gelatinous composition formed and the human serum remained clear. Cloudiness can be seen to have progressed 10 hours after addition (Fig. 13(2)). Cloudiness had progressed even further, although the gelatinous composition remained, 24 hours after addition (Fig. 13(3)). As a result of having carried out thin layer chromatography (TLC) analysis 24 hours after addition, the main eluted components consisted of mono-O-(5,9,13-trimethyltetradeca-4-enoyl)glycerol and (5,9,13-trimethyl-4-tetradecanoic)carboxylate obtained as a result of hydrolysis.

### [Example 10] Evaluation of Blood Flow Inhibitory Effect on Tumor Site Using Emulsion

### (1) Production of Breast Cancer Cell-Transplanted Mouse

4T1 breast cancer cells stably expressing luciferase (4T1-Luc) were acquired and 0.04 ml of a cell suspension containing 2.5 × 10⁷ cells/ml in PBS/Geltrex® (=1/1 (vol/vol) were transplanted beneath the skin of a Balb/c nu/nu nude mouse (Charles River Japan).

### (2) Blood Flow Inhibitory Effect

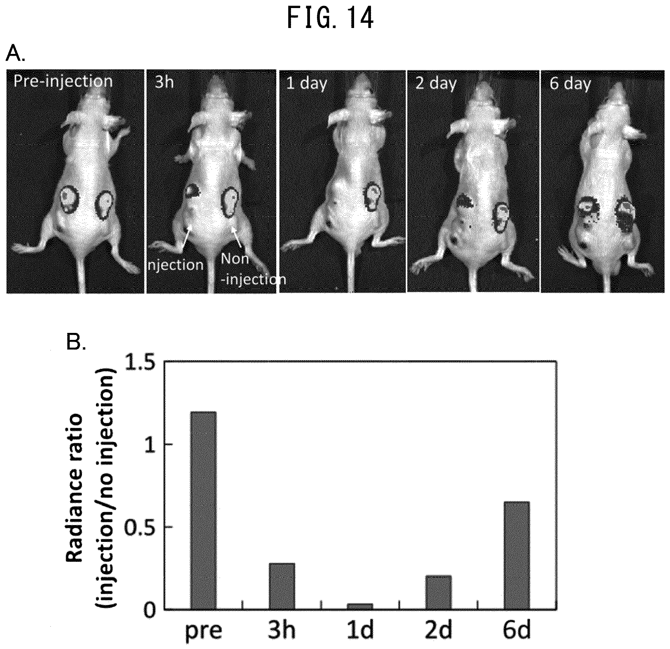

An emulsion prepared using a high-pressure homogenizer was administered locally to the tumor site of the mouse transplanted with the aforementioned breast cancer cells. Following administration, phosphate-buffered physiological saline containing a substrate of luciferase in the form of D-luciferin was administered by intraperitoneal administration and allowed to circulate through the blood followed by observing luciferase (Luc) activity of the aforementioned breast cancer cells over time with an in vivo imaging system (IVIS) (Spectrum). Fluorescence based on Luc activity was confirmed due to the presence of luciferin in the blood. As shown in Fig. 14A, in contrast to being unable to continuously observe fluorescence attributable to expression of Luc by the transplanted breast cancer cells in the mouse not administered the emulsion, since fluorescence decreased immediately after administration in the mouse administered the emulsion, administration of emulsion was suggested to inhibit delivery of D-luciferin present in the blood to the tumor and blood flow to the tumor site was suggested to be inhibited by administration of the emulsion. In addition, the ratios of Luc activity in the mouse administered the emulsion to the mouse not administered the emulsion were calculated for these observed results, and changes over time are shown in Fig. 14B.

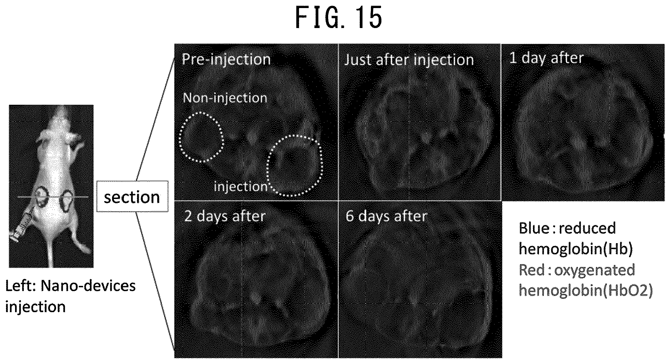

Moreover, the oxygen concentration of intravascular hemoglobin and blood flow at the tumor site were measured by Multispectral Optoacoustic Tomography (MOST) in order to further examine the blood flow inhibitory effect at the tumor site attributable to administration of emulsion. With respect to intravascular hemoglobin, oxygenated hemoglobin is normally higher at measurement sites where blood flow is observed in proportion to oxygen concentration in the blood, while on the other hand, reduced hemoglobin is higher at measurement sites that have become hypoxic. The results of measuring time-based changes in blood oxygen concentration at a tumor site following administration of emulsion are shown in Fig. 15. The signal attributable to reduced hemoglobin (shown in blue in the drawing) increased after administration and reached a maximum after one day. In this manner, since oxygen concentration decreased remarkably at the tumor site administered with the emulsion, this supports the aforementioned blood flow inhibitory effect in which the emulsion is able to block the supply of oxygen and nutrients transported by the blood to tumor tissue.

### (3) Measurement of Blood Flow

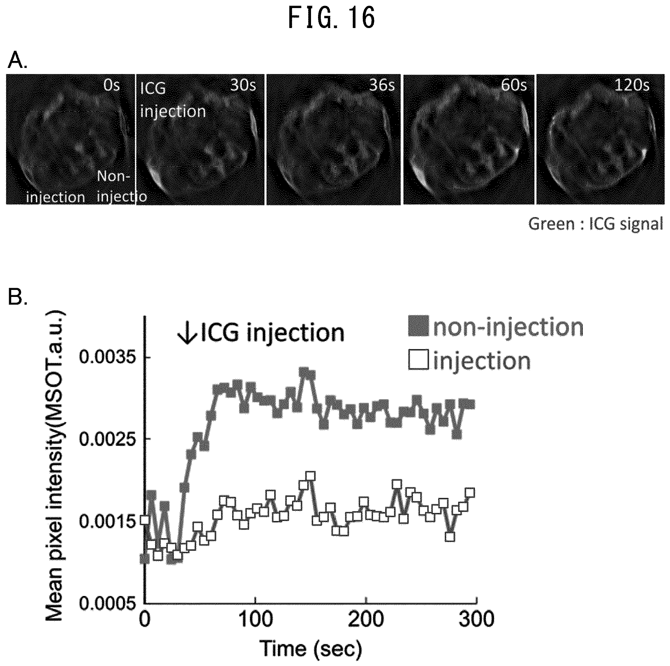

A fluorescent substance in the form of indocyanine green (ICG) was administered intravenously to a tumor-bearing mouse one day after administering the emulsion followed by monitoring blood flow at the tumor site using MSOT (InVision 256, iThera Medical) in order to further examine inhibition of blood flow in a tumor vessel. This type of ICG fluorescent angiography is a method that is used clinically and consists of exciting ICG in the blood bound to plasma protein with infrared light to take advantage of the property of emitting fluorescent light. The results of capturing images of changes in the ICG signal over time are shown in Fig. 16A, while time-based changes in signal strengths corresponding thereto are shown in Fig. 16B. At tumor sites not administered with the emulsion, the ICG signal increased immediately after administration and was maintained for a fixed period of time. On the other hand, at tumor sites where the emulsion was administered, increases in the ICG signal were not observed. On the basis of these results, blood flow in a tumor vessel was suggested to be inhibited in the case of having administered the emulsion to the tumor site.

### (4) Tumor Inhibition

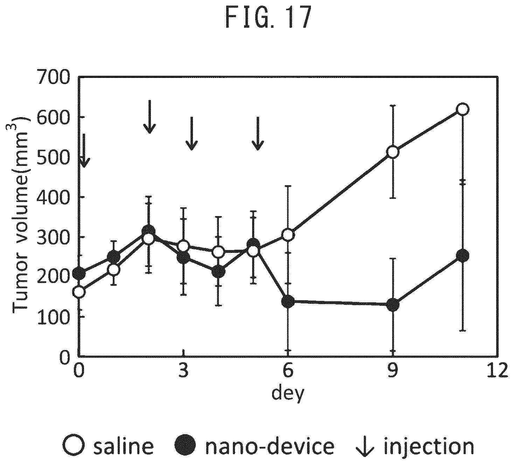

Changes in tumor volume were examined in the case of multiple administrations of the emulsion. Changes in tumor volume were observed over time using a nude mouse transplanted with 4T1-Luc as was described in the aforementioned section (1). The emulsion was administered on initially (day 0) and on days 2, 3 and 5. Time-based changes in tumor volume are shown in Fig. 17. As is clear from Fig. 17, tumor volume decreased in the mouse administered the emulsion in comparison with a control administered physiological saline. The reduction in tumor volume was remarkable in comparison with the case of a single administration (data not shown).

### INDUSTRIAL APPLICABILITY

Use of the microparticles of the tumor sealant of the present invention is expected to provide a greater number of novel choices of lowly invasive treatment methods for preventing cancer metastasis or infiltration. In addition, since the microparticles offer the advantage of being able to be degraded as an embolizing agent while also having a visualization function, open up possibilities for use as a novel embolizing agent for arterial embolization, and are able to contain a contrast agent and/or dye, the microparticles can also be deployed in applications to marking materials during cancer diagnosis and procedures.

All publications and patent literature cited in the present description are incorporated in the present description in their entirety by reference. Furthermore, although the specific embodiments of the present invention have been explained in the present description for the purpose of exemplification, the existence of cases in which the present invention is modified in various ways without deviating from the spirit and scope of the present invention should be easily understood by a person with ordinary skill in the art.

### REFERENCE SIGNS LIST

10 Tumor blood vessel
11 Endothelial cell gap
12 Tumor tissue
13 Microparticle
14 Tumor cell
15 Erythrocyte
20 Emulsion
21 Inverse hexagonal liquid crystal
22 Water channel
23 Outer phase
24 Contrast agent
60 Tumor-bearing mouse
61 Tumor portion
62 Post-administration tumor portion
63 Adhered microparticle
64 Lung

## Claims

1. A biodegradable tumor sealant containing microparticles having for the base material thereof a low molecular weight amphiphilic compound capable of forming non-lamellar liquid crystal.

2. The biodegradable tumor sealant according to claim 1, wherein the microparticles have a particle size of 20 nm to 500 nm.

3. The biodegradable tumor sealant according to claim 1 or 2, wherein the microparticles are present in the form of an emulsion dispersed in an aqueous medium.

4. The biodegradable tumor sealant according to any of claims 1 to 3, wherein the amphiphilic compound is a compound represented by the following general formula (I) or a salt thereof. 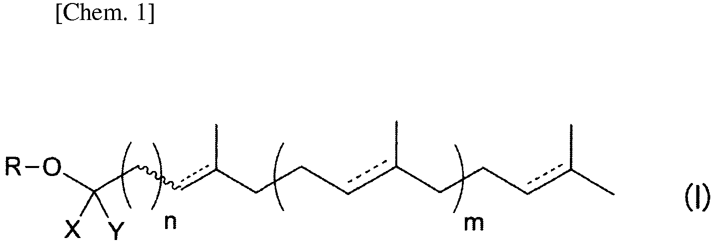 wherein, X and Y respectively represent a hydrogen atom or together represent an oxygen atom, n represents an integer of 0 to 2, and m represents 1 or 2, 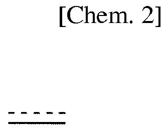 represents a single bond or double bond, and R represents a hydrophilic group having two or more hydroxyl groups.

5. The biodegradable tumor sealant according to claim 4, wherein, in general formula (I), X and Y together represent an oxygen atom, and R represents a hydrophilic group obtained by removing a single hydroxyl group from glycerol.

6. The biodegradable tumor sealant according to any of claims 1 to 5, wherein the amphiphilic compound is any of the following compounds: mono-O-(5,9,13-trimethyltetradecanoyl)glycerol, mono-O-(5,9,13,17-tetramethyloctadecanoyl)glycerol, mono-O-(5,9,13-trimethyltetradeca-4-enoyl)glycerol, mono-O-(5,9,13,17-tetramethyloctadeca-4-enoyl)glycerol, mono-O-(5,9,13-trimethyltetradeca-4,8,12-trienoyl)glycerol or mono-O-(5,9,13,17-tetramethyloctadeca-4,8,12,16-tetraenoyl)glycerol.

7. The biodegradable tumor sealant according to any of claims 1 to 6, wherein the microparticles contain contrast agent and/or dye.

8. The biodegradable tumor sealant according to claim 7, wherein the amphiphilic compound is mono-O-(5,9,13-trimethyltetradeca-4-enoyl)glycerol and the contrast agent is Lipiodol.
